# EUROPÄISCHE PATENTSCHRIFT

(11) **EP 2 147 639 B1**
(45) Veröffentlichungstag und Bekanntmachung des Hinweises auf die Patenterteilung: **17.07.2013**
(21) Anmeldenummer: 09009551.4
(22) Anmeldetag: 23.07.2009
(51) Int. Cl.: A61B 17/02, A61B 17/00

(54) **Wundspreizer mit verstellbaren Valven**
Retractor with adjustable valves
Ecarteurs auto-statiques dotés de valves réglables

(30) Priorität: 25.07.2008 DE 102008034722
(43) Veröffentlichungstag der Anmeldung: 27.01.2010
(73) Patentinhaber: Karl Storz GmbH & Co. KG, 78532 Tuttlingen (DE)
(72) Erfinder: Ott, Ingo, Dr., 64287 Darmstadt (DE); Hermle, Rainer, 78559 Gosheim (DE)
(74) Vertreter: Fugmann, Winfried

(56) Entgegenhaltungen:
- DE-A1- 2 951 664
- DE-U1- 20 119 971
- FR-A- 690 530
- US-A- 3 384 077
- US-A- 3 384 078
- US-A- 5 297 538
- US-A- 5 813 978
- US-A- 6 042 540
- US-A- 6 139 493

## Beschreibung

Die Erfindung liegt auf dem technischen Gebiet der chirurgischen Instrumente und betrifft nach ihrer Gattung einen Wundspreizer mit in ihren Griffbereichen verstellbaren Valven nach dem Oberbegriff von Patentanspruch 1.

Wundspreizer, auch Wundsperrer genannt, dienen dazu, bei einem operativen Eingriff eine Wunde offen zu halten und dem Operateur freien Zugang zum Operationsfeld zu schaffen.

In einer typischen Konstruktionsweise umfassen Wundspreizer zwei gelenkig miteinander verbundene Spreizhebel ("Branchen"), die auf der einen Seite des Gelenks mit Spreizarmen zum Eingriff in eine Wunde und auf dessen anderen Seite mit Betätigungsarmen zur Betätigung des Wundspreizers versehen sind. An den Spreizarmen sind in der Regel flächenförmige Spreizelemente ("Valven") angebracht, die an den Wundrändern verhakt werden können. Über eine manuelle Betätigung der Betätigungsarme, die zu diesem Zweck meist mit Fingereingriffsösen versehen sind, können die beiden Spreizhebel verschwenkt und die Valven in einen wählbaren Relativabstand gebracht werden. Eine lösbare Rastverzahnung dient zur Arretierung der Spreizhebel in der jeweiligen Spreizstellung.

Derartige Wundspreizer werden im Handel in großer Zahl vertrieben, beispielsweise als Wundspreizer nach Weitlaner (DIN 58249), und sind in der Patentliteratur bereits vielfach beschrieben worden.

Nachteilig bei Wundspreizern ist insbesondere die Tatsache, dass die Valven häufig nicht austauschbar sind, so dass für verschiedene Verwendungszwecke, beispielsweise abhängig von der Stärke der zurückzuhaltenden Gewebeschicht, verschiedene Wundspreizer vorgehalten werden müssen. Dies ist mit relativ hohen Anschaffungskosten verbunden. Hinzu kommt, dass sich in manchen Fällen erst beim Einsetzen des Wundspreizers in die Wunde zeigt, welche Valven zum Offenhalten der Wunde am besten geeignet sind. Da Wundspreizer üblicher Weise in symmetrischer Form mit Valven bestückt sind, kann jedoch auch dann, wenn ein Wundspreizer mit in Form und Größe an sich geeigneten Valven in die Wunde eingesetzt wird, ein unbefriedigendes Ergebnis erzielt werden, wenn einander gegenüberliegende Wundränder auf verschiedenen anatomischen Höhen liegen. In diesem Fall ist eine unterschiedliche Arbeitslänge gegenüberliegender Valven erforderlich.

Zur Lösung dieser Probleme zeigt das deutsche Patent Nr. 35 09 787 C2 ein chirurgisches Instrument zum Spreizen von Wundrändern mit Valven deren Griffbereich bzw. Arbeitslänge verstellbar ist. Die Valven bestehen jeweils aus zwei gegeneinander verschiebbaren Teilen, die über einen Spindelmechanismus in verschiedene Relativpositionen verschoben und fixiert werden können. Eine Verstellung des Griffbereichs erfolgt über eine manuelle Drehung der Spindel mit einem Schraubendreher.

Nachteilig bei diesem chirurgischen Instrument ist vor allem die zweiteilige Ausgestaltung der Valven und deren Kopplung über einen Spindelmechanismus, was in der industriellen Serienfertigung relativ hohe Herstellungskosten verursacht. Zudem ist die Verstellung der Arbeitslänge der Valven nur umständlich durch Gebrauch eines Schraubendrehers zu bewerkstelligen, was in nicht unerheblichem Maße Zeit während der Operation in Anspruch nimmt und eine helfende "dritte Hand" erfordern kann. Durch die Verwendung eines Werkzeugs zum Einstellen der Arbeitslänge der Valven und die Möglichkeit hierbei relativ viel Kraft auszuüben, besteht die Gefahr, dass die Valven zu lang eingestellt und in unerwünschter Weise Gewebeschädigungen hervorgerufen werden. Darüber hinaus sind Valven dieser Art nur schwer zu reinigen, da sich insbesondere im Bereich zwischen den beiden Teilen einer Valve und in den Windungen des Spindelmechanismus Verunreinigungen leicht festsetzen können.

Ein weiterer wesentlicher Nachteil dieses Instruments liegt darin, dass die Valven nicht ausgetauscht werden, so dass lediglich die Arbeitslänge der Valven eingestellt werden kann, jedoch keine Möglichkeit besteht, die Form der Valven, insbesondere die Anzahl der Zinken, zu variieren.

Weitere Instrumente dieser Art sind aus der DE 20119971 U1, der US 6,139,493 A und der US 3,384,078 A bekannt. Ihnen gemein ist, dass der Wundspreizer als Rahmenkonstruktion ausgebildet ist und dass die in ihrer Länge einstellbaren Valven aus zwei gegeneinander verschiebbaren Teilen bestehen. Auch hier besteht das Problem, dass sich zwischen den zwei sich überlappenden Teile der Valve Verunreinigungen besonders leicht festsetzen. Auch sind diese Sperrerrahmen nur mit Aufwand und zwei Händen des Benutzers in der Wunde zu platzieren.

Aus der FR 690530 A ist ein Instrument bekannt, dessen Valven jeweils entlang eines Armes eines als Rahmen ausgestalteten Wundspreizers verschiebbar sind, um beispielsweise Operationen am Bein vorzunehmen. Um mit den Armen das Bein zu umfassen und die Valven dann von oben her in der Wunde zu versenken, sind die Valven parallel zu den Armen ausgerichtet.

In der US 5,297,538 A wird ein Wundspreizer mit Fingereingriffsösen und in ihrer Arbeitslänge einstellbaren Valven offenbart (Fig. 23). Hierfür sind die Valven zweiteilig ausgebildet, so dass zur Variation des Greifbereichs die beiden Teile gegeneinander verschoben werden können. Nachteilig ist auch hier, dass der Raum zwischen den beiden in die Wunde eingreifenden Teilen besonders leicht verschmutzt und entsprechend nach Verwendung aufwändig gereinigt werden muss. US 5,297,538 A offenbart den Oberbegriff des Anspruchs 1.

Demgegenüber besteht die Aufgabe der vorliegenden Erfindung darin, einen chirurgischen Wundspreizer zur Verfügung zu stellen, durch welchen die aufgezeigten Nachteile herkömmlicher Wundspreizer mit in ihrem Griffbereich verstellbaren Valven vermieden werden können.

Diese und weitere Aufgaben werden nach dem Vorschlag der Erfindung durch einen Wundspreizer mit den Merkmalen des unabhängigen Patentanspruchs gelöst. Vorteilhafte Ausgestaltungen der Erfindung sind durch die Merkmale der Unteransprüche angegeben.

Ein gattungsgemäßer Wundspreizer umfasst wenigstens zwei Spreizarme, die relativ zueinander verstellbar sind. An ihren Endabschnitten sind die Spreizarme jeweils mit in ihren Greifbereichen verstellbaren, flächenförmigen Spreizelementen (Valven) zum Eingriff in die Wundränder einer Wunde versehen.

Hier und im Weiteren soll unter dem Ausdruck "Greifbereich" einer Valve jener Teil einer Valve verstanden sein, der dem Eingriff in die Wundränder einer Wunde dient und sich somit vom Spreizarm bis zum distalen Ende der Valve, insbesondere deren Zinke(n), erstreckt. Der Greifbereich einer Valve korrespondiert zu deren (wirksamer) Arbeitslänge in Erstreckungsrichtung.

Der erfindungsgemäße Wundspreizer zeichnet sich nun in wesentlicher Weise dadurch aus, dass dieser mit zwei gelenkig verbundenen Spreizhebeln versehen ist, welche jeweils einen auf der einen Seite des Gelenks befindlichen Spreizarm mit einer daran angebrachten Valve zum Eingriff in eine Wunde und einen auf der anderen Seite des Gelenks befindlichen Betätigungsarm mit einer Fingereingriffsöse zur Betätigung des Wundspreizers aufweisen. Wenigstens eine der Valven ist einstückig ausgebildet und in einer von einem Spreizarm geformten Linearführung linear verschiebbar in deren Erstreckungsrichtung so aufgenommen, dass sie mittels einer lösbaren Befestigungseinrichtung in einer Mehrzahl (zumindest zwei) verschiedener Stellungen, die jeweils verschiedenen Greifbereichen (d. h. verschiedenen Arbeitslängen) entsprechen, wahlfrei festlegbar ist, wobei die wenigstens eine Valve in Erstreckungsrichtung im Wesentlichen rechtwinklig zur Längsrichtung des Endabschnitts des Spreizarms ausgerichtet ist und in der Erstreckungsrichtung der Valve verschiebbar gelagert ist. Erfindungsgemäß kann somit in vorteilhafter Weise ohne die Notwendigkeit einer Änderung der tatsächlichen Valvenlänge, wie dies bei den eingangs genannten verstellbaren zweiteiligen Valven der Fall ist, die Arbeitslänge bzw. der zum Eingreifen in eine Wunde zur Verfügung stehende Greifbereich verändert und an die jeweilige Situation angepasst werden.

Bei einer besonders vorteilhaften Ausgestaltung des erfindungsgemäßen Wundspreizers ist die Linearführung in Form einer in Führungsrichtung beidseitig offenen Schwalbenschwanzführung ausgebildet, wobei die Valve wenigstens in einem darin aufgenommenen Abschnitt eine hierzu komplementäre Passform aufweist. Schwalbenschwanzführungen bieten den besonderen Vorteil, dass die darin aufgenommenen Valven unter Belastung so verkanten, dass die Festlegung der Valven in Führungsrichtung durch die Befestigungseinrichtung unterstützt wird.

Bei einer weiteren vorteilhaften Ausgestaltung des erfindungsgemäßen Wundspreizers weist die lösbare Befestigungseinrichtung wenigstens ein Eingriffspaar auf, das ein Eingriffsmittel umfasst, das kraft- und/oder formschlüssig in eine zugehörige Ausnehmung greift. Hierbei ist das Eingriffsmittel mit einem aus Spreizarm und daran angebrachter Valve gewählten Element verbunden und an dem jeweils anderen Element sind eine Mehrzahl Ausnehmungen zum Eingriff mit dem Eingriffsmittel geformt. Besonders vorteilhaft ist hierdurch ein Rastmechanismus realisiert. Das Eingriffsmittel, welches beispielweise in Kugelform ausgebildet ist, kann insbesondere durch ein Federmittel in kraft- und/oder formschlüssigen Eingriff mit jeweils einer der Ausnehmungen, die beispielsweise kreisförmig oder kugelschalenförmig ausgebildet sind, gebracht werden. Vorteilhaft sind die Ausnehmungen beispielsweise mit einem gleichen Zwischenabstand an den Valven in einer Reihe entlang deren Erstreckungsrichtungen ausgebildet.

Das Eingriffsmittel kann beispielsweise auch in Form eines Gewindeelements ausgebildet sein, das jeweils mit einer der Ausnehmungen in kraft- und/oder formschlüssigen Eingriff bringbar ist.

Bei einer weiteren vorteilhaften Ausgestaltung des erfindungsgemäßen Wundspreizers sind die zwei relativ zueinander verstellbaren Spreizarme durch eine Sperrvorrichtung in einer Verstellposition wahlfrei arretierbar.

Die Erfindung wird nun anhand eines Ausführungsbeispiels näher erläutert, wobei Bezug auf die beigefügten Zeichnungen genommen wird. Gleiche bzw. gleichwirkende Elemente sind in den Zeichnungen mit denselben Bezugszahlen bezeichnet. Es zeigen:
- Fig. 1: in einer perspektivischen Ansicht ein Ausführungsbeispiel des erfindungsgemäßen Wundspreizers;
- Fig. 2: in einer weiteren perspektivischen Ansicht einen Spreizarm des Wundspreizers von Fig. 1;
- Fig. 3: in einer Schnittansicht den Spreizarm von Fig. 2 gemäß Schnittlinie D-D;
- Fig. 4: in einer weiteren Schnittansicht den Spreizarm von Fig. 2 gemäß Schnittlinie G-G von Fig. 3;
- Fig. 5: in einer weiteren perspektivischen Ansicht den Wundspreizer von Fig. 1 mit geschlossenen Spreizarmen;
- Fig. 6: in einer weiteren perspektivischen Ansicht den Wundspreizer von Fig. 1 mit geöffneten Spreizarmen und Valven mit maximalem Greifbereich;
- Fig. 7: in einer weiteren perspektivischen Ansicht den Wundspreizer von Fig. 1 mit geöffneten Spreizarmen und Valven mit minimalem Greifbereich.

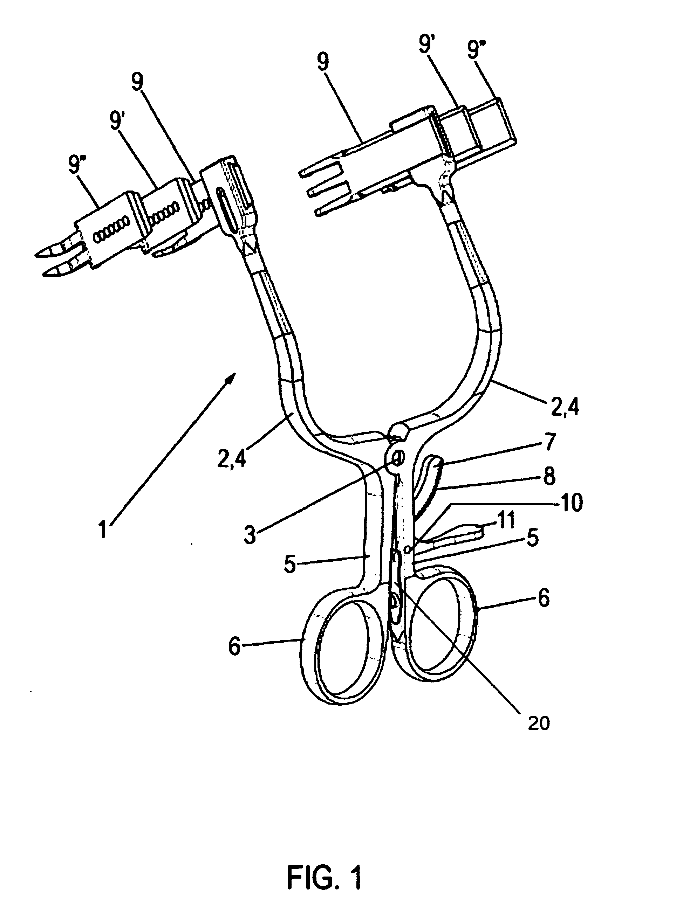

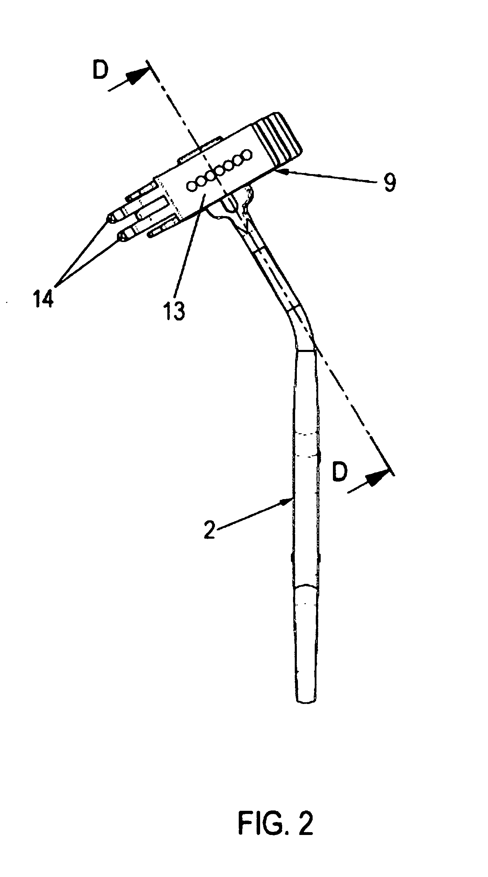

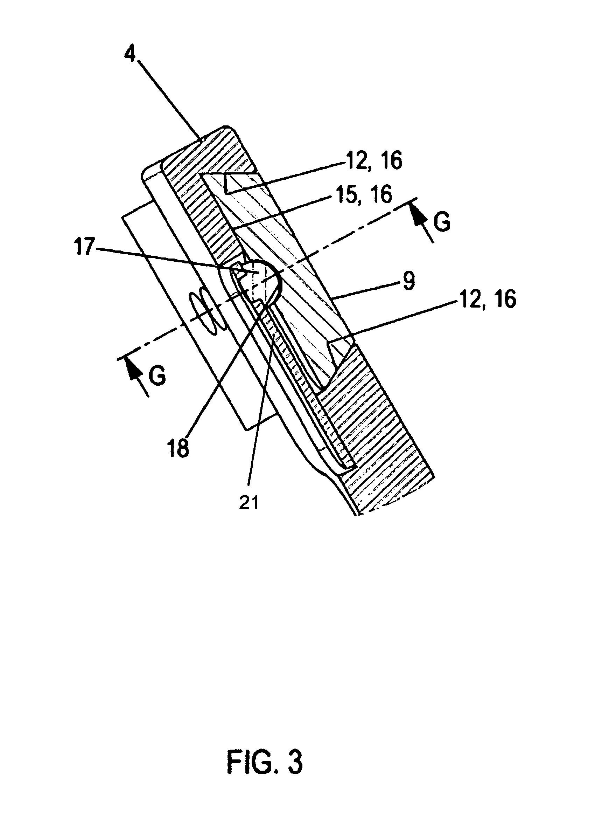

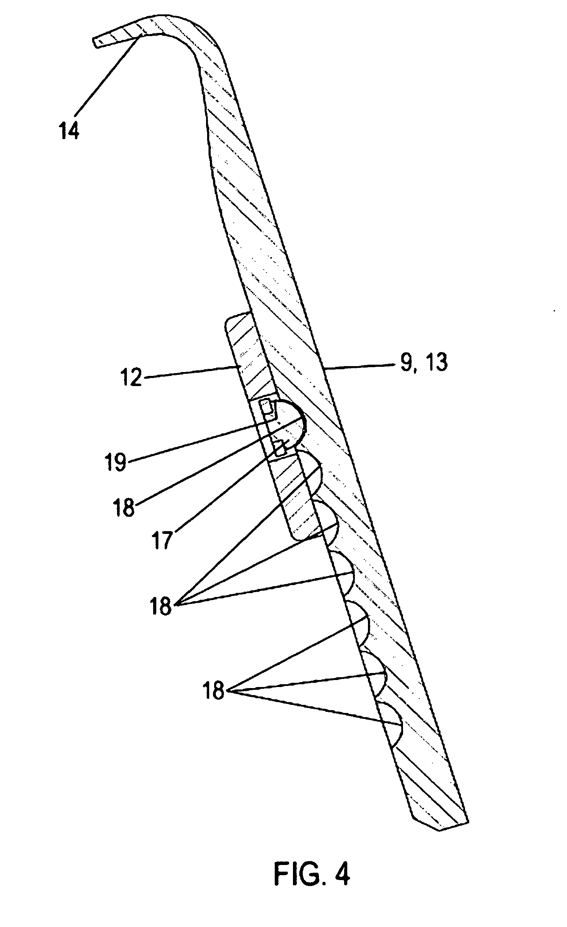

Unter Bezugnahme auf Fig. 1 bis Fig. 4 wird nun ein Ausführungsbeispiel eines insgesamt mit der Bezugszahl 1 bezeichneten erfindungsgemäßen Wundspreizers beschrieben.

Demnach umfasst der Wundspreizer 1 zwei Spreizhebel 2, die etwa in deren Mitte über ein Scharniergelenk 3 gelenkig miteinander verbunden sind. Jeder der beiden Spreizhebel 2 umfasst einen auf der einen Seite des Scharniergelenks 3 befindlichen Spreizarm 4 und einen auf der anderen Seite des Scharniergelenks 3 befindlichen Betätigungsarm 5. Die Endabschnitte der beiden Betätigungsarme 5 sind jeweils mit Fingereingriffsösen 6 versehen, die dazu dienen, die Spreizhebel 2 manuell zu bewegen und die Spreizarme 4 in verschiedene Schwenk- bzw. Spreizstellungen zu bringen.

Über eine in Form einer Rastverzahnung ausgebildete Sperrvorrichtung können die Spreizhebel 2 in einer wahlfreien Schwenkstellung in der die beiden Valven 9 einen gewünschten Relativabstand voneinander einnehmen, verrastet werden. Die Rastverzahnung umfasst eine an einem der beiden Betätigungsarme angebrachte, bogenförmige Lasche 7 mit einer entlang des Bogens verlaufenden Zahnung 8 und eine in Fig. 1 verdeckte Kinke, die durch eine Feder 20 in Eingriff mit der Zahnung 8 gehalten wird. Die Kinke ist an einen Fingerhebel 11 angeformt, der an dem anderen Betätigungsarm mittels eines Gelenkstifts 10 angelenkt ist. Die Zähne der Zahnung 8 sind so geformt, dass die Kinke in der Art eines Ratschenmechanismus beim Zusammenführen der beiden Fingereingriffsösen 6 über die Zahnung 8 gleiten kann, jedoch in der umgekehrten Bewegungsrichtung in den Zahnlücken verhakt und somit ein Zusammenführen der Spreizarme 4 sperrt. Durch Betätigen des Fingerhebels 11 entgegen der Federkraft der die Kinke in Eingriff mit der Zahnung 8 haltenden Feder kann der Rastmechanismus gelöst werden, um die beiden Spreizarme 4 zueinander zu führen.

An den Endabschnitten der beiden Spreizarme 4 sind jeweils einstückige Valven 9 zum Eingreifen in gegenüberliegende Wundränder angebracht. Die Valven 9 bestehen jeweils aus einem flächigen Flächenabschnitt 13 und in Erstreckungsrichtung der Valven 9 vom Flächenabschnitt 13 abstehende gekrümmte Zinken 14. Die Valven 9 sind an den Endabschnitten der beiden Spreizarme 4 in Erstreckungsrichtung verschiebbar gelagert und zu diesem Zweck jeweils mit ihrem Flächenabschnitt 13 in einer an den Endabschnitten der Spreizarme 4 geformten Schwalbenschwanzführung 12 aufgenommen. Jede Schwalbenschwanzführung 12 ist im Querschnitt quer zur Erstreckungsrichtung der Valven 9 in der Art eines Schwalbenschwanzes geformt, d. h. sie umfasst eine in etwa fluchtend zur Erstreckungsrichtung des Endabschnitts des zugehörigen Spreizarms 4 angeordnete Basisfläche 15 und zwei in einem spitzen Winkel (Winkel geringer als 90°) zur Basisfläche 15 angestellte, konisch konvergierende Seitenflächen 16, welche die darin aufgenommene Valve 9 von drei Seiten umgeben. Die beiden Schwalbenschwanzführungen 12 sind hierbei so angeordnet, dass sie sich jeweils zur anderen Schwalbenschwanzführung 12 hin konisch verjüngen. Die Valven 9 weisen im Bereich des Flächenabschnitts 13 eine komplementäre Passform zur Schwalbenschwanzführung 12 auf, wobei die Flächenabschnitte 13 der Basisfläche 15 anliegen. Die Schwalbenschwanzführungen 12 ermöglichen eine Linearverschiebung der Valven 9 entlang deren Erstreckungsrichtungen und legen die Valven 9 in den anderen Richtungen fest.

Eine Fixierung der Valven 9 erfolgt jeweils durch einen Rastmechanismus, welcher eine innerhalb der Schwalbenschwanzführung 12 in einer Kugelaufnahme 19 aufgenommene Rastkugel 17 umfasst, die durch die Federkraft des Federmittels 21 in Eingriff mit einer im Flächenabschnitt 13 geformten Ausnehmung 18 der Valve 9 gebracht wird. Die Flächenabschnitte 13 jeder Valve 9 sind zu diesem Zweck jeweils mit einer Mehrzahl Ausnehmungen 18 versehen, die in Erstreckungsrichtung der Valve 9 mit einem in etwa gleichen Zwischenabstand verteilt angeordnet sind. Die kugelschalenförmigen Ausnehmungen 18 weisen eine korrespondierende Passform zur Rastkugel 17 auf. Die Rastkugel 17 und eine in Eingriff mit der Rastkugel 17 befindliche Ausnehmung 18 formen gemeinsam ein Eingriffspaar.

Durch den Rastmechanismus kann jede Valve 9 in verschiedenen Raststellungen entsprechend dem jeweils in Eingriff befindlichen Eingriffspaar entlang deren Erstreckungsrichtung innerhalb der Schwalbenschwanzführung 12 fixiert werden. Durch Verändern der Raststellung kann hierbei der Greifbereich bzw. die Arbeitslänge einer Valve 9, welcher durch den Abstand des distalen Endes der Valve 9 (bzw. deren Zinken 14) vom zugehörigen Spreizarm 4 gegeben ist, eingestellt werden. Durch die in Erstreckungsrichtung beidseitig offenen Schwalbenschwanzführungen 12 können die Valven 9 in Raststellungen mit einem gegenüber dem maximalen Greifbereich verkürztem Greifbereich aus der Schwalbenschwanzführung 12 ragen. Eine Verstellung der Valven 9 innerhalb der Schwalbenschwanzführungen 12 erfolgt manuell, wobei der Rastmechanismus einerseits eine gute Verschiebbarkeit und andererseits einen sicheren Halt gewährleistet. Bei in Arbeitsstellung gebrachtem Wundspreizer 1 wird durch die Wundränder eine Kraft auf die Valven 9 ausgeübt, durch die die Valven 9 in Richtung zueinander belastet werden. Dies hat in besonders vorteilhafter Weise eine Verkantung der Valven 9 in den Schwalbenschwanzführungen 12 zu Folge, durch welche die Fixierung der Valven 9 unterstützt wird.

In den Figuren sind die Valven 9 beispielhaft mit sieben mit einem jeweils gleichen Zwischenabstand in einer Reihe entlang der Verschiebungs- bzw. Erstreckungsrichtung der Valven 9 angeordneten Ausnehmungen 18 (entsprechend sieben verschiedener Raststufen) versehen. Lediglich der Vollständigkeit sei darauf hingewiesen, dass auch eine größere oder kleinere Anzahl von Raststufen möglich ist.

Denkbar wäre auch, dass anstelle einer federbelasteten Rastkugel 17 eine manuell verstellbare Schraube vorgesehen ist, welche jeweils in kraft- und/oder formschlüssigen Eingriff mit den im Flächenabschnitt 13 geformten Ausnehmungen 18 gebracht werden kann.

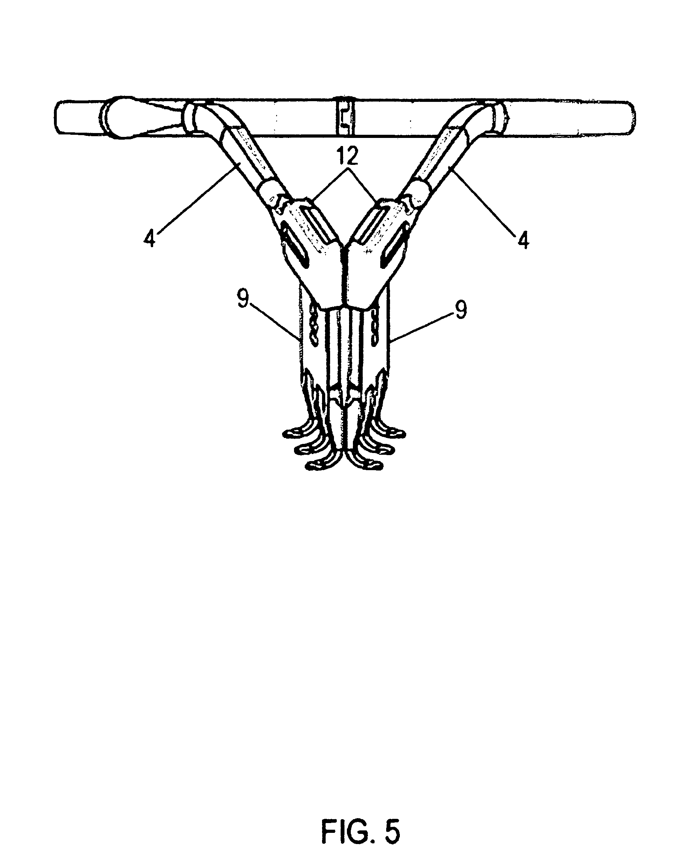

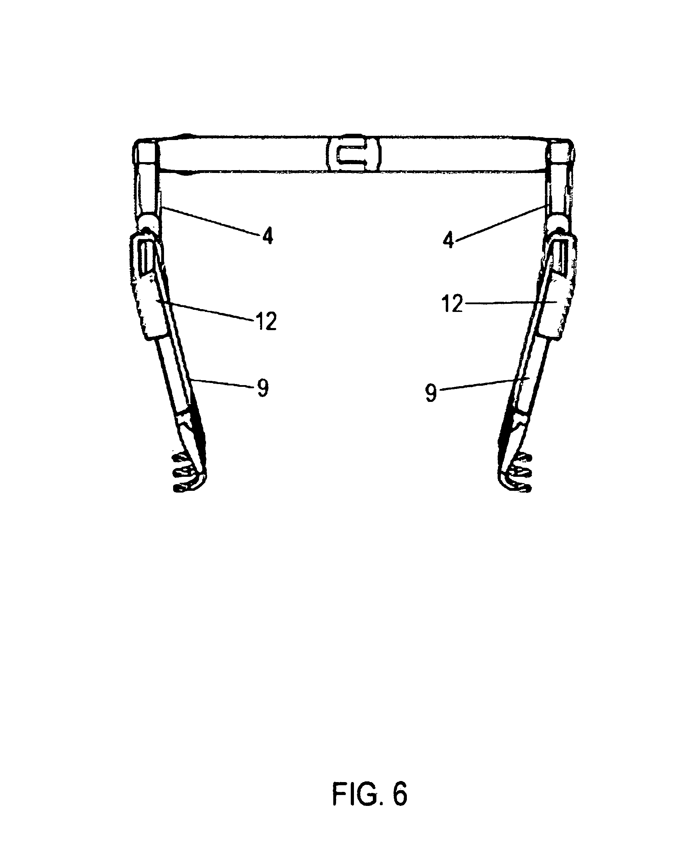

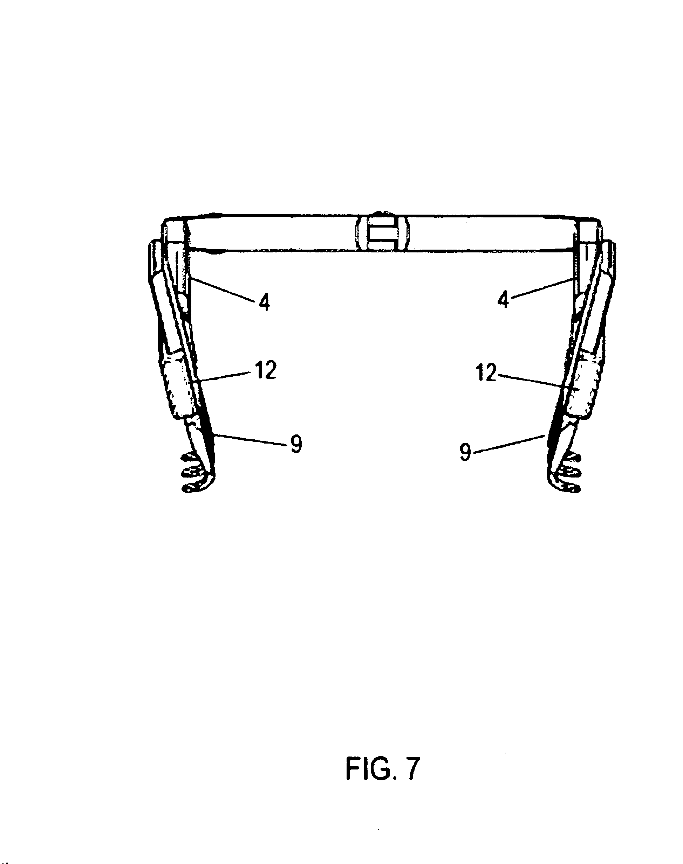

In den Fig. 5 bis 7 sind verschiedene Stellungen der beiden Spreizhebel 2 gezeigt. Fig. 5 zeigt eine Situation, bei der die beiden Spreizarme 4 einander berühren, entsprechend einem maximalen Abstand der beiden Fingereingriffsösen 6. In Fig. 6 sind die beiden Spreizarme 4 durch Zusammenbringen der Fingereinriffsösen 6 in eine maximale Spreizstellung gebracht worden. Die in Erstreckungsrichtung geringfügig angestellten Valven 9 haben hier eine maximale Arbeitslänge (maximaler Greifbereich), d. h. die Rastkugel 17 greift in jene Ausnehmung 18, die von den Zinken 14 am weitesten beabstandet ist. Demgegenüber zeigt Fig. 7 eine Situation, bei der die beiden Valven 9 eine minimale Arbeitslänge (minimaler Greifbereich) haben, d. h. die Rastkugel 17 greift in jene Ausnehmung 18, die den geringsten Abstand zu den Zinken 14 hat. Entsprechend den verschiedenen Raststufen kann die Arbeitslänge der einstückigen Valven 9 zwischen der minimalen bzw. maximalen Arbeitslänge variiert werden. Obgleich dies in den Figuren nicht dargestellt ist, können für die beiden Valven 9 wahlfrei auch voneinander verschiedene Arbeitslängen eingestellt werden, insbesondere um den Wundspreizer 1 in Wunden mit in unterschiedlichen anatomischen Höhen befindlichen Wundrändern einzusetzen.

Aufgrund der in Führungsrichtung beidseitig offenen Schwalbenschwanzführungen 12 können die Valven 9 sehr einfach ausgetauscht werden, um je nach Verwendungszweck verschieden große bzw. unterschiedlich geformte Valven einzusetzen. In Fig. 1 sind beispielhaft weitere Valven 9', 9" gezeigt, die sich in der Anzahl ihrer Zinken unterscheiden.

### Bezugszeichenliste

- 1: Wundspreizer
- 2: Spreizhebel
- 3: Scharniergelenk
- 4: Spreizarm
- 5: Betätigungsarm
- 6: Fingereingriffsöse
- 7: Lasche
- 8: Zahnung
- 9, 9', 9": Valve
- 10: Gelenkstift
- 11: Fingerhebel
- 12: Schwalbenschwanzführung
- 13: Flächenabschnitt
- 14: Zinke
- 15: Basisfläche
- 16: Seitenfläche
- 17: Rastkugel
- 18: Ausnehmung
- 19: Kugelaufnahme
- 20: Feder
- 21: Federmittel

## Patentansprüche

1. Wundspreizer (1) mit wenigstens zwei Spreizarmen (4), die relativ zueinander verstellbar sind und an ihren Endabschnitten jeweils mit in ihren Greifbereichen verstellbaren Valven (9) zum Eingriff in eine Wunde versehen sind, wobei der Wundspreizer mit zwei gelenkig verbundenen Spreizhebeln (2) versehen ist, welche jeweils einen der auf der einen Seite des Gelenks (3) befindlichen Spreizarme (4) mit daran angebrachter valve (9) zum Eingriff in eine Wunde und einen auf der anderen Seite des Gelenks (3) befindlichen Betätigungsarm (5) mit Eingereingriffsöse (6) zur netätigung des Wundspreizers aufweisen, **dadurch gekennzeichnet, dass** wenigstens eine der Valven (9) einstückig ausgebildet ist und in einer von einem Spreizarm (4) geformten Linearführung (12) linear verschiebbar so aufgenommen ist, dass sie mittels einer lösbaren Befestigungseinrichtung (17, 18) in einer Mehrzahl verschiedener Stellungen, die jeweils verschiedenen Greifbereichen entsprechen, wahlfrei festlegbar ist, wobei die wenigstens eine Valve in Erstreckungsrichtung im Wesentlichen rechtwinklig zur Längsrichtung des Endabschnitts des Spreizarms (4) ausgerichtet ist und in der Erstreckungsrichtung der Valve verschiebbar gelagert ist.

2. Wundspreizer (1) nach Anspruch 1, **dadurch gekennzeichnet, dass** die Linearführung in Form einer in Führungsrichtung beidseitig offenen Schwalbenschwanzführung (12) ausgebildet ist und die Valve (9) wenigstens in einem darin aufgenommenen Abschnitt (13) eine hierzu komplementäre Passform aufweist.

3. Wundspreizer (1) nach einem der Ansprüche 1 bis 2, **dadurch gekennzeichnet, dass** die lösbare Befestigungseinrichtung wenigstens ein Eingriffspaar (17, 18) aufweist, welches ein kraft- und/oder formschlüssig im eine Ausnehmung (18) greifendes Eingriffsmittel (17) umfasst, wobei das Eingriffsmittel (17) mit einem aus Spreizarm (4) und zugehöriger Valve (9) gewählten Element verbunden ist und in dem jeweils anderen Element eine Mehrzahl Ausnehmungen (18) zum Eingriff mit dem Eingriffsmittel geformt sind.

4. Wundspreizer (1) nach Anspruch 3, **dadurch gekennzeichnet, dass** die lösbare Befestigungseinrichtung (17, 18) einen Rastmechanismus formt.

5. Wundspreizer (1) nach Anspruch 4, **dadurch gekennzeichnet, dass** das Eingriffsmittel (17) durch ein Federmittel in kraft-und/oder formschlüssigen Eingriff mit einer aus der Mehrzahl Ausnehmungen (18) gebracht wird.

6. Wundspreizer (1) nach Anspruch 5, **dadurch gekennzeichnet, dass** das Eingriffsmittel (17) kugelförmig und die Ausnehmungen (18) jeweils kreisförmig oder kugelschalenförmig ausgebildet sind.

7. Wundspreizer (1) nach einem der Ansprüche 3 bis 6, **dadurch gekennzeichnet, dass** die Ausnehmungen (18) an den Valven (9) reihenförmig entlang deren Erstreckungsrichtungen angeordnet sind.

8. Wundspreizer (1) nach Anspruch 3, **dadurch gekennzeichnet, dass** das Eingriffsmittel (17) in Form eines mit jeweils einer der Ausnehmungen (18) in kraft- und/oder formschlüssigen Eingriff bringbaren Gewindeelements ausgebildet ist.

9. Wundspreizer (1) nach einem der vorhergehenden Ansprüche, **dadurch gekennzeichnet, dass** die Spreizhebel (2) durch eine lösbare Sperrvorrichtung (7,8) arretierbar sind.

## Claims

1. Retractor (1) with at least two spreader arms (4), which are adjustable relative to each other and which, at their end sections, are each provided with blades (9) adjustable in their grip areas for engagement in a wound, wherein the retractor is provided with two spreader levers (2), which are connected in an articulated manner and each have one of the spreader arms (4), located on one side of the joint (3), with a blade (9) mounted thereon for engagement in a wound and, on the other side of the joint (3), an actuating arm (5) with finger eyelet (6) for the actuation of the retractor, **characterized in that** at least one of the blades (9) is formed in one piece and is received in a linearly displaceable manner in a linear guide (12) formed by a spreader arm (4), in such a way that it can be fixed optionally, by means of a releasable securing device (17, 18), in a plurality of different positions, which each correspond to various grip areas, wherein the at least one blade is oriented, in the direction of extent thereof, substantially at right angles to the longitudinal direction of the end section of the spreader arm (4) and is mounted displaceably in the direction of extent of the blade.

2. Retractor (1) according to Claim 1, **characterized in that** the linear guide is designed in the form of a dovetail guide (12) open on both sides in the guide direction, and the blade (9), at least in a section (13) received therein, has a complementary form-fit shape.

3. Retractor (1) according to one of Claims 1 to 2, **characterized in that** the releasable securing device has at least one engaging pair (17, 18), which comprises an engaging means (17) that engages with a force fit and/or form fit in a recess (18), wherein the engaging means (17) is connected to one element chosen from spreader arm (4) and associated blade (9), and a plurality of recesses (18) for engagement with the engaging means are formed on the respective other element.

4. Retractor (1) according to Claim 3, **characterized in that** the releasable securing device (17, 18) forms a catch mechanism.

5. Retractor (1) according to Claim 4, **characterized in that** the engaging means (17) is brought into force-fit and/or form-fit engagement with one of the plurality of recesses (18) by a spring means.

6. Retractor (1) according to Claim 5, **characterized in that** the engaging means (17) are spherical, and the recesses (18) are each circular or cup-shaped.

7. Retractor (1) according to one of Claims 3 to 6, **characterized in that** the recesses (18) on the blades (9) are arranged in rows along the direction of extent thereof.

8. Retractor (1) according to Claim 3, **characterized in that** the engaging means (17) is designed in the form of a threaded element that can be brought into force-fit and/or form-fit engagement with one of the recesses (18) in each case.

9. Retractor (1) according to one of the preceding claims, **characterized in that** the spreader levers (2) can be locked by a releasable locking device (7, 8).

## Revendications

1. Écarteur autostatique (1) comprenant au moins deux bras d'écartement (4), qui peuvent être déplacés l'un par rapport à l'autre et qui sont munis, au niveau de leurs sections d'extrémité, de valves respectives (9) déplaçables dans leurs régions d'engagement pour s'engager dans un champ opératoire, l'écarteur autostatique étant muni de deux leviers d'écartement (2) connectés de manière articulée, lesquels présentent à chaque fois l'un des bras d'écartement (4) situés sur un côté de l'articulation (3) avec une valve (9) montée sur celui-ci, pour s'engager dans un champ opératoire et un bras d'actionnement (5) situé de l'autre côté de l'articulation (3) avec un oeillet d'engagement pour les doigts (6) pour l'actionnement de l'écarteur autostatique, **caractérisé en ce qu'**au moins l'une des valves (9) est réalisée d'une seule pièce et est reçue de manière déplaçable linéairement dans un guide linéaire (12) formé par un bras d'écartement (4), de telle sorte qu'elle puisse être fixée de manière librement sélective au moyen d'un dispositif de fixation desserrable (17, 18) dans une pluralité de positions différentes, qui correspondent à chaque fois à différentes régions d'engagement, l'au moins une valve étant orientée dans la direction d'étendue essentiellement à angle droit par rapport à la direction longitudinale de la section d'extrémité du bras d'écartement (4) et étant montée de manière déplaçable dans la direction d'étendue de la valve.

2. Écarteur autostatique (1) selon la revendication 1, **caractérisé en ce que** le guide linéaire est réalisé sous forme d'un guide en queue d'aronde (12) ouvert des deux côtés dans une direction de guidage et la valve (9) présente au moins dans une section (13) reçue dans celle-ci une forme adaptée complémentaire à celle-ci.

3. Écarteur autostatique (1) selon l'une quelconque des revendications 1 à 2, **caractérisé en ce que** le dispositif de fixation desserrable présente au moins une paire d'engagement (17, 18) qui comprend un moyen d'engagement (17) s'engageant par force et/ou par coopération de forme dans un évidement (18), le moyen d'engagement (17) étant connecté à un élément sélectionné parmi le bras d'écartement (4) et la valve associée (9) et une pluralité d'évidements (18) pour l'engagement avec le moyen d'engagement étant formée sur l'autre élément respectif.

4. Écarteur autostatique (1) selon la revendication 3, **caractérisé en ce que** le dispositif de fixation desserrable (17, 18) forme un mécanisme à encliquetage.

5. Écarteur autostatique (1) selon la revendication 4, **caractérisé en ce que** le moyen d'engagement (17) est amené par un moyen de ressort en engagement par force et/ou par coopération de forme avec l'un de la pluralité d'évidements (18).

6. Écarteur autostatique (1) selon la revendication 5, **caractérisé en ce que** le moyen d'engagement (17) est réalisé sous forme sphérique et les évidements (18) sont respectivement réalisés sous forme circulaire ou sous forme de coque sphérique.

7. Écarteur autostatique (1) selon l'une quelconque des revendications 3 à 6, **caractérisé en ce que** les évidements (18) sur les valves (9) sont disposés en rangées le long de leur directions d'étendue.

8. Écarteur autostatique (1) selon la revendication 3, **caractérisé en ce que** le moyen d'engagement (17) est réalisé sous forme d'un élément fileté pouvant être amené en engagement par force et/ou par coopération de forme avec à chaque fois l'un des évidements (18).

9. Écarteur autostatique (1) selon l'une quelconque des revendications précédentes, **caractérisé en ce que** les leviers d'écartement (2) peuvent être bloqués par un dispositif de blocage desserrable (7, 8).
